# EUROPÄISCHE PATENTSCHRIFT

(11) **EP 1 147 075 B1**
(45) Veröffentlichungstag und Bekanntmachung des Hinweises auf die Patenterteilung: **10.09.2003**
(21) Anmeldenummer: 00904895.0
(22) Anmeldetag: 11.01.2000
(51) Int. Cl.: C07C 213/00, C07C 215/60

(54) **VERFAHREN ZUR HERSTELLUNG VON L-PHENYLEPHRINHYDROCHLORID**
METHOD FOR PRODUCING L-PHENYLEPHRINE HYDROCHLORIDE
PROCEDE DE PREPARATION D'HYDROCHLORURE DE L-PHENYLEPHRINE

(30) Priorität: 21.01.1999 DE 19902229
(43) Veröffentlichungstag der Anmeldung: 24.10.2001
(73) Patentinhaber: Boehringer Ingelheim Pharma GmbH & Co.KG, 55218 Ingelheim am Rhein (DE)
(72) Erfinder: KLINGLER, Franz, Dietrich, D-64347 Griesheim (DE); WOLTER, Lienhard, D-55606 Hochstetten/Dhaun (DE); DIETRICH, Wolfgang, D-55543 Bad Kreuznach (DE)
(74) Vertreter: Laudien, Dieter, Dr.
(86) Internationale Anmeldenummer: EP0000144
(87) Internationale Veröffentlichungsnummer: WO00043345

(56) Entgegenhaltungen:
- SAKURABA S ET AL: "EFFICIENT ASYMMETRIC HYDROGENATION OF ALPHA-AMINO KETONE DERIVATIVES A HIGHLY ENANTIOSELECTIVE SYNTHESIS OF PHENYLEPHRINE, LEVAMISOLE, CARNITINE AND PROPRANOLOL" CHEMICAL AND PHARMACEUTICAL BULLETIN,JP,PHARMACEUTICAL SOCIETY OF JAPAN. TOKYO, Bd. 43, Nr. 5, 1. Mai 1995 (1995-05-01), Seiten 738-747, XP000571426 ISSN: 0009-2363 in der Anmeldung erwähnt

## Beschreibung

Die vorliegende Erfindung betrifft ein verbessertes Verfahren zur Herstellung von L-Phenylephrinhydrochlorid mittels Rhodium-katalysierter asymmetrischer Hydrierung im industriellen Maßstab.

### Technologischer Hintergrund der Erfindung

L-Phenylephrin gehört zu den pharmazeutisch häufig eingesetzten Analoga des Andrenalins und ist von hohem kommerziellem Interesse. L-Phenylephrin wird pharmazeutisch als L-Phenylephrinhydrochlorid eingesetzt und wirkt als Sympathomimetikum in der Therapie der Hypotonie und als Vasokonstringens in der Augen- und Nasenheilkunde. Die chemische Struktur des chiralen α-Aminoalkohols L-Phenylephrin ist in der Formel I dargestellt. Formel I:

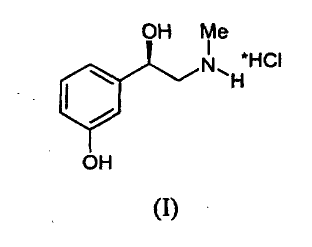

### Stand der Technik

Zu den aus dem Stand der Technik bekannten Herstellungsverfahren von L-Phenylephrinhydrochlorid gehört die asymmetrische Hydrierung des prochiralen N-Benzyl-N-methyl-2-amino-m-benzyloxyacetophenonhydrochlorids (Formel II) nach Tetrahedron Letters 30 (1989), 367 - 370, bzw. Chem. Pharm. Bull. 43 (5) (1995) 738 - 747. Formel II:

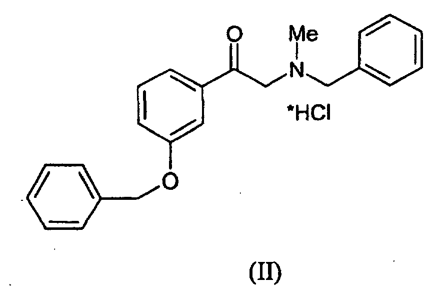

Achiwa et al. beschreiben in Tetrahedron Letters 30 (1989), 367- 370 die asymmetrische Hydrierung von 3-BenzyIoxy-2-(N-benzyl-N-methyl)-aminoacetophenonhydrochlorid als Substrat mit Wasserstoff in Gegenwart von [Rh(COD)Cl]₂ /(2R, 4R)-4-(Dicyclohexylphosphino)-2-(diphenylphosphino-methyl)-N-methyl-aminopyrrolidin als Katalysator. Unmittelbar nach Filtration und Einengung des Reaktionsgemischs wird die benzylische Stickstoff-Schutzgruppe abgespalten und Phenylephrin als Produkt erhalten. Dabei fällt neben dem L-Enantiomeren das D-Enantiomere mit einem Anteil von mindestens. 7,5% als Verunreinigung an (85% ee). Für die Reaktion muß der Katalysator bezogen auf das Substrat in einem Mol-Verhältnis von 1 : 2000 eingesetzt werden. Der Nachteil dieses Verfahrens besteht im wesentlichen darin, daß das erhaltene L-Phenylephrin nicht in wirtschaftlicher Weise auf eine für die Verwendung als Arzneimittel erforderliche Reinheit von mindestens 98% ee aufgereinigt werden kann.

In Chem. Pharm. Bull. 43 (5) (1995) 738 - 747 wird für die asymmetrische Hydrierung ein Mol-Verhältnis von Substrat zu Katalysator von etwa 1000 : 1 als bevorzugt angegeben.

Zur Herstellung von L-Phenylephrin in industriellem Maßstab ist jedoch das im Stand der Technik beschriebene Verfahren aufgrund mehrere Nachteile nicht geeignet: Das Produkt ist trotz der Verwendung großer Mengen Katalysator im asymmetrischen Reaktionsschritt als L-Enantiomeres für pharmazeutische Zwecke ohne aufwendige Reinigungsprozeduren nicht ausreichend rein herstellbar, sondern nur als Mischung mit einem relativ hohem Anteil an D-Enantiomeren als Verunreinigung zugänglich.
Auch stellt die relativ lange Reaktionszeit des asymmetrischen Hydrierungsschritts von ca. 20 Stunden gerade für die Herstellung von L-Phenylephrin in industriellen Maßstab einen apperativ sehr aufwendigen und teueren Reaktionsschritt mit einem nicht zu vernachlässigenden Sicherheitsrisko dar.

### Beschreibung der Erfindung

Die vorliegende Erfindung betrifft ein neues Herstellungsverfahren für L-Phenylephrinhydrochlorid durch asymmetrische Hydrierung unter Überwindungen der aus dem Stand der Technik bekannten bzw. der oben aufgeführten Schwierigkeiten und Nachteile.

Eines der wesentlichen Ziele der vorliegenden Erfindung ist es, ein Verfahren zu entwickeln, durch das L-Phenylephrinhydrochlorid in hoher optischer und chemischer Reinheit hergestellt werden kann. Damit soll z.B. die Gefahr einer Verunreinigung von Arzneistoffen, die L-Phenylephrinhydrochlorid als Wirkstoff enthalten, mit dem unerwünschten D-Enantiomeren minimiert werden.

Ein weiteres Ziel der Erfindung besteht darin, ein Verfahren zu entwickeln, durch das weitgehend enantiomerenreines L-Phenylephrin in einfacher Weise dargestellt werden kann.

Ein weiteres Ziel der Erfindung besteht darin, L-Phenylephrin über ein stereoselektives Verfahren herzustellen, um Reaktionsschritte zu vermeiden, bei denen chirale Zwischenverbindungen bzw. das chirale Endprodukt L-Phenylephrin als Racemat in einer ähnlichen Menge wie der entsprechende Antipode anfällt.

Ein weiteres Ziel des erfindungsgemäßen Verfahrens ist es, die für die Herstellung von L-Phenylephrinhydrochlorid notwendigen Hydrierungszeiten deutlich zu verringern, um die u.a. mit der Verwendung von unter hohem Druck stehendem Wasserstoff verbundenen Kosten und Gefahren zu reduzieren.

Ein weiteres Ziel der vorliegenden Erfindung ist es , dem Fachmann ein Herstellverfahren für L-Phenylephrin an die Hand zu geben, das diesen in großen Mengen benötigten Wirkstoff ausgehend von einfach zugänglichen Edukten auf kostengünstige Weise zugänglich macht.

Überraschenderweise wurde nun gefunden, daß L-Phenylephrinhydrochlorid in außergewöhnlich hoher optischer Reinheit aus N-Benzyl-N-methyl-2-amino-mhydroxyacetophenonhydrochlorid 1 unter Anwendung einer asymmetrischen Hydrierung mit [Rh(COD)Cl]₂/(2R, 4R)-4-Dicyclohexylphosphino)-2-(diphenylphosphino-methyl)-N-methyl-aminocarbonylpyrrolidin als Katalysatorsystem und einer speziellen Abfolge von Folgeschritten zugänglich ist. Die in der Summenformel benutzte Abkürzung COD steht für Cyclooctadien.

Bei einem Mol-Verhältnis Katalysator zu Substrat von ca. 1:1000 erhält man durch das erfindungsgemäße Verfahren ausgehend von Benzyladrianon (N-Benzyl-N-methyl-2-aminom-hydroxy-acetophenon-hydrochlorid) **1** Benzyladrianolhydrochlorid **2** bereits in einer optischen Reinheit von 92% ee (Reaktionsschema 1). Durch Überführen des Benzyladrianolhydrochlorid **2** in die freie Base und anschließendem Ausfällen derselben aus einem Amoniak-Methanol-Wasser-Gemisch kann die optische Reinheit in einfacher und bemerkenswerter Weise sogar auf > 99% ee gesteigert werden. Diese für pharmazeutische Zwecke ausreichend optisch reine Zwischenverbindung wird dann in einem anschließenden Reaktionsschritt in das L-Phenylephrinhydrochlorid **3** überführt.

Der genaue Mechanismus der Rhodium-katalysierten asymmetrischen Hydrierung ist bisher nicht bekannt. Das gilt insbesondere für die Umsetzung von N-Benzyl-N-methyl-2-amino-mhydroxyacetophenonhydrochlorid **1** mit Wasserstoff unter Katalyse von [Rh(COD)Cl]₂ und (2R, 4R)-4-(Dicyclohexylphosphino)-2-(diphenylphosphino-methyl)-N-methylaminocarbonylpyrrolidin als Katalysatorsystem.

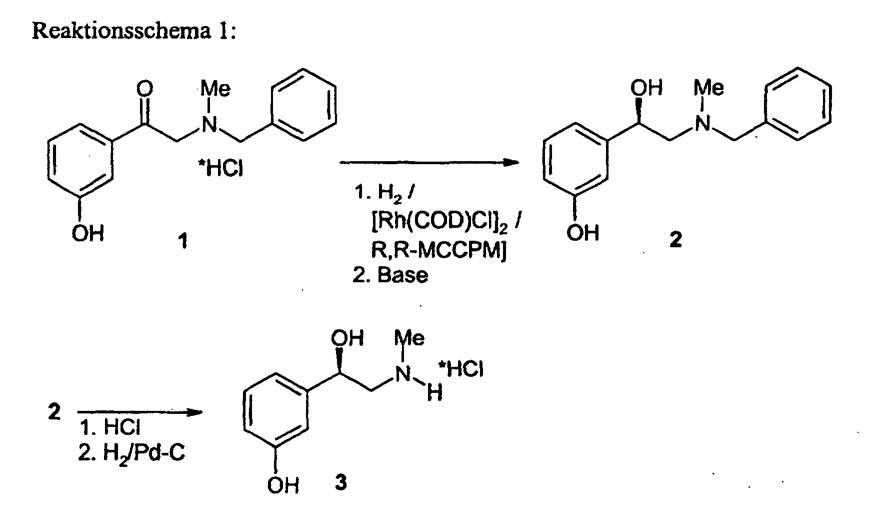

Weiter wurde gefunden, daß entgegen der herrschenden Meinung für den asymmetrischen Hydrierungsschritt zum Erzielen guter Ausbeuten bzw. hoher optischer Reinheit ein Mol-Verhältnis von Katalysator zu Substrat nicht - wie durch den Stand der Technik nahegelegt von etwa 1:1000 notwendig ist. Im erfindungsgemäßen Verfahren kann dieses Verhältnis drastisch um den Faktor 10 bis 100 gesenkt werden. Trotz dieser signifikanten Verringerung der Katalysatormenge wird das aus der asymmetrischen Hydrierung hervorgehende Zwischenprodukt **2** - und damit letztlich L-Phenylephrin - dennoch in einer deutlich höheren optischen Ausbeute erhalten als durch das aus dem Stand der Technik bekannten Verfahren. So wird beispielsweise L-Phenylephrin bei einer Katalysatorkonzentration von lediglich 1:10000 noch in einer opt. Ausbeute von 88% ee erhalten. Durch die Reduktion der Katalystormenge wird die Aufreinigung des Produktes deutlich vereinfacht.

Durch die Verringerung der Katalysatormenge und Verwendung des kommerziell günstigen N-Benzyl-N-methyl-2-amino-m-hydroxyacetophenon **1** als Edukt können die Kosten der Herstellung von L-Phenylephrin durch das neue Verfahren deutlich gesenkt werden.

Zusätzlich konnte durch das neue Verfahren die Reaktionszeit der asymmetrischen Hydrierung um bis zu 75% gegenüber dem Stand der Technik reduziert werden. Letzteres ist gerade unter Kosten- und Sicherheitsgesichtspunkten für die Herstellung von L-Phenylephrin in industriellem Maßstab besonders vorteilhaft.

Schließlich ist es gemäß dem erfindungsgemäßen Verfahren möglich, auf den Schutz der phenolischen Hydroxylgruppe in dem 2-Aminoketon **1** zu verzichten und dabei **1** trotzdem erfolgreich zu dem chiralen 2-Aminoalkohol **2** unter Anwendung einer asymmetrischen Hydrierung mit einem der erfindungsgemäßen Katalysatorsysteme umzusetzen.

Daneben ermöglicht das erfindungsgemäße Verfahren durch die Reinigung auf der Stufe des Benzyladrianols **2** die Gewinnung des L-Phenylephrins in hoher optischer Reinheit.

Gemäß Reaktionsschema 1 wird das kommerziell günstige N-Benzyl-N-methyl-2-amino-mhydroxyacetophenon **1** in einem ersten Reaktionsschritt in Gegenwart eines chiralen Rhodiumkatalysators mit Wasserstoff unter einem Druck in einem Bereich von 10 bis 100 bar, vorzugsweise 10 bis 50 bar und ganz besonders bevorzugt bei 20 bar, zum N-Benzyl-L-Phenylephrinhydrochlorid **2** umgesetzt.

Als Katalysator wird erfindungsgemäß [Rh(COD)Cl]₂ und ein chiraler, zweizähniger Phosphinligand eingesetzt. Bevorzugt wird (2R, 4R)-4-(Dicyclohexylphosphino)-2-(diphenylphosphino-methyl)-N-methyl-aminocarbonylpyrrolidin (RR-MCCPM) als Katalysator eingesetzt.
Die Herstellung dieses Katalysators ist aus dem Stand der Technik bekannt [EP-A-0 251 164, EP-A-O 336 123]. Der Katalysator kann auch polymergebunden vorliegen, z.B. indem der chirale Ligand (2R, 4R)-4-Dicyclohexylphosphino)-2-(diphenylphosphino-methyl)-N-methylaminocarbonyl) pyrrolidin z.B. über die Phenylgruppen an ein Polymer gebunden ist. Dabei schließt die Verwendung solcher polymergebundener Liganden den gleichzeitigen Einsatz von nicht-polymergebundenem Liganden nicht zwingend aus. Solche polymergebunden Katalysatoren sind insbesondere für eine einfache Reinigung des Produktes von Vorteil.

Der Katalysator wird entweder als vorgefertigte, sauerstofffreie Lösung von [Rh(COD)Cl]₂ und Ligand eingesetzt oder *in situ* aus [Rh(COD)Cl]₂ und Ligand in Gegenwart des N-Benzyl-N-methyl-2-amino-m-hydroxyacetophenonhydrochlorids **1** sauerstofffrei unter Schutzgasatmosphäre oder Wasserstoffatmosphäre hergestellt.

Das Mol-Verhältnis von N-Benzyl-N-methyl-2-amino-m-hydroxyacetophenonhydrochlorid **1** und Katalysator liegt im erfindungsgemäßen Verfahren zwischen 5000:1 und 100000:1, bevorzugt zwischen 5000:1 und 20000:1 und besonders bevorzugt bei ca. 10000:1.

Die Hydrierung wird bei einer Reaktionstemperatur von ca. 40 bis 100°C durchgeführt. Der bevorzugte Temperaturbereich liegt zwischen 40 und 60° C, besonders bevorzugt sind Temperaturen in einem Intervall von 50 - 55°C.

Als Reaktionsmedien können sowohl protische Lösungsmittel - wie z.B. Alkohole und/oder Wasser - oder aprotische polare Lösungsmittel wie z.B. Ether und/oder Amide bzw. Lactame und/oder Gemische davon eingesetzt werden. Allen Lösungsmitteln kann gegebenenfalls Wasser zugesetzt sein. Als protische Lösungsmittel werden bevorzugt verzweigte oder unverzweigte C₁ - C₈ Alkanole eingesetzt. Besonders bevorzugt werden niedere Alkohole wie Methanol, Ethanol, n-Propanol und Isopropanol oder deren Mischungen eingesetzt. Besonders bevorzugt wird als Reaktionsmedium Methanol verwendet, wobei das Methanol oder die anderen Alkohole oder Lösungsmittel gegebenenfalls Wasser enthalten kann (können). Als aprotische Lösungsmittel eignen sich polare Ether wie beispielsweise Tetrahydrofuran oder Dimethoxyethylether oder Amide wie beispielsweise Dimethylformamid, oder Lactame wie beispielsweise N-Methylpyrrolidon. Bevorzugt werden Lösungsmittel eingesetzt, die wenig zur Brennbarkeit neigen.

Da das Edukt **1** als Hydrochlorid vorliegt, wird es durch Zusatz einer Base *in situ* zunächst in die freie Base überführt, um die Löslichkeit zu erhöhen. Als Base können organische Basen oder anorganische Basen sowohl als Feststoffe als auch in Form von Lösungen eingesetzt werden, z.B. als wäßrige Lösungen. Als anorganische Basen eignen sich basisch reagierende Alkalisalze oder Alkalihydroxyde. Bevorzugt werden Alkalihydrogencarbonate oder Alkalicarbonate neben Alaklihydroxiden eingesetzt. Ganz besonders bevorzugt werden Na₂CO₃, K₂CO₃, LiOH, NaOH, KOH, NaHCO₃ verwendet. Die Erfindung schließt auch die Verwendung anderer basisch reagierender Stoffe oder weitere Stoffe, die das Hydrochlorid **1** in die freie Base überführen können und aus dem Stand der Technik bekannt sind, mit ein.

Als organische Basen eignen sich besonders tert. Alkyl-Amine oder tert. Alkyl-Aryl-Amine. Bevorzugt werden Trialkylamine mit verzweigten oder unverzweigten C₁ - C₅-Alkylresten eingesetzt. Als ganz besonders bevorzugt haben sich beispielsweise Triethylamin oder Diisopropylethylamin bewährt. Gegebenenfalls kann die Reaktion auch in Gegenwart von basischen Polymeren mit z.B. tert. Aminofunktionen durchgeführt werden.

Die asymmetrische Hydrierung wird bei einem Druck zwischen mehr als 1 bar und maximal 100 bar, bevorzugt zwischen 10 und 50 bar, und besonders bevorzugt bei ca. 20 bar durchgeführt.

Die Reaktion wird sauerstofffrei, zweckmäßigerweise unter Inertgas, durchgeführt, bevorzugt unter Wasserstoffatmosphäre. Für die Reaktion ist es jedoch nicht zwingend, daß der Wasserstoff für die Hydrierung aus dem Atmosphärengas über der Reaktionsmischung entnommen werden kann. Der Wasserstoff kann auch *in situ* in Lösung aus geeigneten Wasserstoffquellen erzeugt werden. Zu solchen Wasserstoffquellen zählen z.B. Ammoniumformiat, Ameisensäure und andere Formiate, Hydrazine in Gegenwart von Metallionen wie Fe²⁺/Fe³⁺ und andere aus dem Stand der Technik bekannte Wasserstoffquellen.

Die Reaktionszeit für die asymmetrische Hydrierung beträgt bis zu ihrer Beendigung zwischen 2 und 8 Stunden, bevorzugt liegt sie zwischen 4 und 6 Stunden, besonders bevorzugt beträgt sie 4 Stunden.

Die Umsetzung von N-Benzyl-L-phenylephrin **2** zum Phenylephrinhydrochlorid **3** gelingt durch Palladium-katalysierte hydrierende Debenzylierung. Dabei kann die Reaktionsmischung der asymmetrischen Hydrierung ohne weitere Aufarbeitung mit einem Palladium-Katalysator versetzt werden (Methode A).

Bei dieser Methode wird die Reaktionslösung der asymmetrischen Hydrierung unmittelbar nach der Reaktion mit Aktivkohle und einer Palladiumchlorid-Lösung versetzt und unter einem Druck von größer 1 bis 5 bar, bevorzugt sei 2 - 3 bar, hydriert. Die weitere Aufarbeitung erfolgt nach literaturbekannten Verfahren.

Bevorzugt wird jedoch aus der Reaktionslösung der asymmetrischen Hydrierung zunächst N-Benzyl-L-phenylephrin **2** durch einfache Aufarbeitung und Kristallisation als Rohprodukt isoliert und danach in Lösung der Palladium-katalysierten Debenzylierung mit Wasserstoff unter Druck zugeführt (Methode B, siehe Beispiele). Überraschenderweise hat sich nämlich gezeigt, daß die nach der asymmetrischen Hydrierung notwendige Enantiomerentrennung auf der Stufe des N-Benzyl-L-Phenylephrin **2** deutlich erfolgreicher und einfacher durchführbar ist als auf der Stufe des L-Phenylephrins bzw. des Hydrochlorids **3**.

Das erfindungsgemäße Verfahren soll nun durch die nachfolgenden Beispiele erläutert werden. Dem Fachmann ist bewußt, daß die Beispiele nur zur Veranschaulichung dienen und als nicht limitierend anzusehen sind.

### Beispiele

### Herstellung der Katalysator-Lösung:

In 2 l entgastes Methanol werden unter Schutzgas 4,3 g Dichloro-bis-[cycloocta-1,5-dien)rhodium (I)] und 9,4 g RR-MCCPM (2R,4R)-4-(Dicyclohexyl-phosphino)-2-(diphenylphosphino-methyl)- N-methyl-aminocarbonylpyrrolidin eingetragen und 30 min. bei Raumtemperatur gerührt.

### Asym. Hydrierung von N-Benzyl-N-methyl-2-amino-m-hydroxyacetophenonhydrochlorid 1 zu N-Benzyl-L-Phenylephrin 2:

In einem 500 l Autoklav 80 kg N-Benzyl-N-methyl-2-amino-mhydroxyacetophenonhydrochlorid **1**, 0,58 kg Triethylamin und 240 l Methanol vorgelegt, entgast und mit obiger Katalysatorlösung versetzt. Anschließend wird auf 50 - 55°C erwärmt und durch Wasserstoff wird ein Druck von 20 bar erzeugt. Nach ca. 4 h ist die vollständige Hydrierung erfolgt.

### Weitere Umsetzung von N-Benzyl-L-phenylephrin 2 zu L-Phenylephrinhydrochlorid 3:

### Methode A:

Die oben genannte Hydrierlösung wird in einem zweiten 500 l Rührkessel mit 0,8 kg Aktivkohle und ca. 69 g Palladium als Palladiumchlorid-Lösung versetzt und bei 2 bar H₂-Druck hydriert. Nach erfolgter Reaktion wird das Lösungsmittelgemisch i. Vak. abdestilliert und mit ca. 80 l Wasser versetzt. Danach wird mit 50 %iger Kaliumcarbonatlösung bei ca. 65°C ein pH von ca. 9,5 eingestellt und die Lösung auf 10 - 15°C abgekühlt. Der kristalline Niederschlag wird abgetrennt und mit ca. 100 l H₂O gewaschen. Die Rohbase wird in ca. 120 I Wasser eingetragen, mit konzentrierter Salzsäure (ca. 18 l) auf einen pH von ca. 6,5 eingestellt und auf 50 - 60°C erwärmt. Die Lösung wird mit Aktivkohle (2,4 kg) versetzt und filtriert. Danach wird ein pH von 2,5 - 3,0 eingestellt und der Hauptteil des Wassers i. Vak. abdestilliert. Der Rückstand wird in zunächst in ca. 145 l Isopropanol gelöst. Danach wird auf ca. 100 l eingeengt und auf 10 - 15 °C gekühlt. Das auskristallisierte L-Phenylephrinhydrochlorid 3 wird abgetrennt und durch Schleudern und Trocknen von Isopropanol befreit. Man erhält L-Phenylephrinhydrochlorid **3** in einer Ausbeute von ca. 40 kg (ca. 71 % bezogen auf N-Benzyl-N-methyl-2-amino-m-hydroxyacetophenonhydrochlorid **1**) und in einer chemischen Reinheit von > 99 % und einer optischen Reinheit von > 96 % ee.

### Methode B:

Die oben beschriebene Hydrierlösung wird i. Vak. abdestilliert, mit 118 l Wasser versetzt, auf 50 - 60° C erwärmt und mit Aktivkohle versetzt. Nach Abtrennen der Kohle werden ca. 801 Wasser und 235 l Methanol zugegeben und auf 35 - 45°C erwärmt. Danach wird die Lösung mit ca. 57 l konzentrierter Ammoniak-Lösung versetzt und auf ca. 15 - 25° C gekühlt. Der entstehende kristalline Niederschlag wird abgetrennt, mit ca. 100 l Wasser gewaschen und getrocknet. Man erhält ca. 57 kg N-Benzyl-N-methyl-2-amino-m-hydroxyacetophenon als freie Base. Diese wird mit ca. 114 l Wasser, 18 l konzentrierter Salzsäure (pH ca. 5,5 - 6,5) und ca. 57 g Palladium als Palladium-Kohle versetzt und bei 55° - 65°C bei 2 bar H₂-Druck hydriert. Nach erfolgter Reaktion (1 - 2 h) wird das entstandene Toluol azeotrop mit Wasser abdestilliert. Danach wird die Lösung mit Aktivkohle versetzt, filtriert und auf einen pH von 3,4 - 3,6 eingestellt, bevor man ca. 110 l Wasser abdestilliert. Der Rückstand wird in ca. 150 l Isopropanol aufgenommen und auf 15 - 20°C gekühlt. Das auskristallisierte Produkt wird abgetrennt und getrocknet. Nach Trocknen erhält man ca. 38 kg L-Phenylephrinhydrochlorid 3. Die Mutterlauge wird im Vakuum bis zum Rückstand abdestilliert, in ca. 201 Wasser aufgenommen, mit konzentriert Salzsäure auf einen pH von 6,2 - 6,5 eingestellt, mit Aktivkohle versetzt, filtriert und schließlich auf einen pH von 3,4 - 3,6 eingestellt. Danach wird das Lösungsmittel destillativ entfernt, der Rückstand in ca. 15 l Isopropanol gelöst und erneut kristallisiert. Nach Abtrennung und Trocknen erhält man ca. 4,5 kg L-Phenylephrinhydrochlorid **3**. Die Gesamtausbeute an **3** beträgt ca. 42,5 kg (76% bezogen auf N-Benzyl-N-methyl-2-amino-m-hydroxyacetophenonhdrochlorid **1**). Die chemische Reinheit ist > 99 % und die optischen Reinheit ist > 99 % ee.

## Patentansprüche

1. Herstellungsverfahren von L-Phenylephrinhydrochlorid **3, dadurch gekennzeichnet, daß** man ausgehend von prochiralem N-Benzyl-N-methyl-2-amino-mhydroxyacetophenonhydrochlorid **1** als Edukt
in einem ersten Schritt
a. eine asymmetrische Hydrierung mit [Rh(COD)Cl]₂ und einen chiralen, zweizähnigen Phosphinliganden als Katalysatorsystem
und in einem zweiten Reaktionsschritt
b. eine reduktive Debenzylierung mit Palladium und Wasserstoff durchführt.

2. Verfahren nach Anspruch 1, **dadurch gekennzeichnet daß** der Ligand (2R, 4R)-4-(Dicyclohexylphosphino)-2-(diphenylphosphino-methyl)-N-methyl-aminocarbonylpyrrolidin ist.

3. Verfahren nach Anspruch 1, **dadurch gekennzeichnet daß** der Ligand polymergebundenes (2R, 4R)-4-(Dicyclohexylphosphino)-2-(diphenylphosphino-methyl)-N-methylaminocarbonyl-pyrrolidin ist.

4. Verfahren nach einem der Ansprüche 1 bis 3, **dadurch gekennzeichnet, daß** die asymmetrische Hydrierung in einem Temperaturbereich von 40°C bis 100°C durchgeführt wird.

5. Verfahren nach Anspruch 4, **dadurch gekennzeichnet, daß** die asymmetrische Hydrierung in einem Temperaturbereich von 40°C bis 60°C durchgeführt wird.

6. Verfahren nach Anspruch 5, **dadurch gekennzeichnet, daß** die asymmetrische Hydrierung in einem Temperaturbereich von 50°C bis 55°C durchgeführt wird.

7. Verfahren nach einem der vorangegangenen Ansprüche 1 bis 6, **dadurch gekennzeichnet, daß** die asymmetrische Hydrierung unter einem Druck von mehr als 1 bar bis 100 bar durchgeführt wird.

8. Verfahren nach Anspruch 7, **dadurch gekennzeichnet, daß** die asymmetrische Hydrierung unter einem Druck von 10 bar bis 50 bar durchgeführt wird.

9. Verfahren nach Anspruch 8, **dadurch gekennzeichnet, daß** die asymmetrische Hydrierung unter einem Druck von 20 bar durchgeführt wird.

10. Verfahren nach Anspruch 9, **dadurch gekennzeichnet, daß** die asymmetrische Hydrierung in einem protischen Lösungsmittel durchgeführt wird.

11. Verfahren nach einem der vorangegangenen Ansprüche 1 bis 10, **dadurch gekennzeichnet, daß** die asymmetrische Hydrierung in einem verzweigten oder unverzweigten C₁ - C₈-Alkanol als Lösungsmittel durchgeführt wird.

12. Verfahren nach Anspruch 11, **dadurch gekennzeichnet, daß** die asymmetrische Hydrierung in Methanol, Ethanol, n-Propanol und/oder Isopropanol als Lösungsmittel durchgeführt wird.

13. Verfahren nach einem der vorangegangenen Ansprüche 10 bis 12, **dadurch gekennzeichnet, daß** das Lösungsmittel für die asymmetrische Hydrierung Wasser enthält.

14. Verfahren nach einem der vorangegangenen Ansprüche 1 bis 13, **dadurch gekennzeichnet, daß** das Mol-Verhältnis N-Benzyl-N-methyl-2-amino-mhydroxyacetophenonhydrochlorid **1** zum Rhodiumkatalysator in der asymmetrischen Hydrierung zwischen 5000:1 und 100000:1 beträgt.

15. Verfahren nach dem vorangegangenen Anspruch 14, **dadurch gekennzeichnet, daß** das Mol-Verhältnis N-Benzyl-N-methyl-2-amino-m-hydroxyacetophenonhydrochlorid **1** zum Rhodiumkatalysator in der asymmetrischen Hydrierung zwischen 5000:1 und 20000:1 beträgt.

16. Verfahren nach Anspruch 15, **dadurch gekennzeichnet, daß** das Mol-Verhältnis N-Benzyl-N-methyl-2-amino-m-hydroxyacetophenonhydrochlorid **1** zum Rhodiumkatalysator in der asymmetrischen Hydrierung 10000:1 beträgt.

17. Verfahren nach einem der vorangegangenen Ansprüche 1 bis 16, **dadurch gekennzeichnet, daß** der Rhodiumkatalysator für die asymmetrische Hydrierung als vorgefertigte Lösung eingesetzt wird.

18. Verfahren nach einem der vorangegangenen Ansprüche 1 bis 16, **dadurch gekennzeichnet, daß** der Rhodiumkatalysator für die asymmetrische Hydrierung *in situ* erzeugt wird.

19. Verfahren nach einem der vorangegangenen Ansprüche 1 bis 18, **dadurch gekennzeichnet, daß** die Reaktionszeit für die asymmetrische Hydrierung zwischen 2 und 8 Stunden liegt.

20. Verfahren nach dem vorangegangenen Anspruch 19, **dadurch gekennzeichnet, daß** die Reaktionszeit für die asymmetrische Hydrierung zwischen 4 und 6 Stunden liegt.

21. Verfahren nach Anspruch 20, **dadurch gekennzeichnet, daß** die Reaktionszeit für die asymmetrische Hydrierung 4 Stunden beträgt

22. Verfahren nach einem der vorangegangenen Ansprüche 1 bis 21, **dadurch gekennzeichnet, daß** die Reaktionslösung der asymmetrischen Hydrierung nach Schritt a des Anspruchs 1 nach beendeter Reaktion ohne Aufarbeitung mit Aktivkohle und einer Palladiumchloridlösung versetzt und mit Wasserstoff einem Druck von 2 bar ausgesetzt wird und anschließend das Reaktionsprodukt isoliert wird.

23. Verfahren nach einem der vorangegangenen Ansprüche 1 bis 21, **dadurch gekennzeichnet, daß** nach beendeter asymmetrischen Hydrierung nach dem Reaktionsschritt a des Anspruchs 1 das entstandene N-Benzyl-L-Phenylephrin **2** als Rohprodukt isoliert wird, dieses anschließend in Wasser bei einem pH-Wert in einem Bereich von 5-7 gelöst und mit Palladiumkohle versetzt wird, die Lösung danach mit Wasserstoff einem Druck von 1 bis 5 bar ausgesetzt wird und schließlich das Reaktionsprodukt isoliert wird.

## Claims

1. Method of preparing L-phenylephrine hydrochloride 3, **characterised in that**, starting from prochiral N-benzyl-N-methyl-2-amino-m-hydroxyacetophenone hydrochloride 1 as educt,
in a first step
a. asymmetric hydrogenation is carried out with [Rh(COD)Cl]₂ and a chiral, bidentate phosphine ligand as the catalyst system
and in a second reaction step
b. reductive debenzylation is carried out with palladium and hydrogen.

2. Process according to claim 1, **characterised in that** the ligand is (2R, 4R)-4-(dicyclohexylphosphino)-2-(diphenylphosphino-methyl)-N-methylaminocarbonyl-pyrrolidine.

3. Process according to claim 1, **characterised in that** the ligand is polymer-bound (2R, 4R)-4-(dicyclohexylphosphino)-2-(diphenylphosphinomethyl)-N-methyl-aminocarbonyl-pyrrolidine.

4. Process according to one of claims 1 to 3, **characterised in that** the asymmetric hydrogenation is carried out in a temperature range of from 40°C to 100°C.

5. Process according to claim 4, **characterised in that** the asymmetric hydrogenation is carried out in a temperature range of from 40°C to 60°C.

6. Process according to claim 5, **characterised in that** the asymmetric hydrogenation is carried out in a temperature range of from 50°C to 55°C.

7. Process according to one of the preceding claims 1 to 6, **characterised in that** the asymmetric hydrogenation is carried out under a pressure of more than 1 bar up to 100 bar.

8. Process according to claim 7, **characterised in that** the asymmetric hydrogenation is carried out under a pressure of 10 bar to 50 bar.

9. Process according to claim 8, **characterised in that** the asymmetric hydrogenation is carried out under a pressure of 20 bar.

10. Process according to claim 9, **characterised in that** the asymmetric hydrogenation is carried out in a protic solvent.

11. Process according to one of the preceding claims 1 to 10, **characterised in that** the asymmetric hydrogenation is carried out in a branched or unbranched C₁ - C₈-alkanol as the solvent.

12. Process according to claim 11, **characterised in that** the asymmetric hydrogenation is carried out in methanol, ethanol, n-propanol and/or isopropanol as solvent.

13. Process according to one of the preceding claims 10 to 12, **characterised in that** the solvent contains water for the asymmetric hydrogenation.

14. Process according to one of the preceding claims 1 to 13, **characterised in that** the molar ratio of N-benzyl-N-methyl-2-amino-m-hydroxyacetophenone hydrochloride **1** to the rhodium catalyst in the asymmetric hydrogenation is between 5,000:1 and 100,000:1.

15. Process according to the preceding claim 14, **characterised in that** the molar ratio of N-benzyl-N-methyl-2-amino-m-hydroxyacetophenone hydrochloride **1** to the rhodium catalyst in the asymmetric hydrogenation is between 5,000:1 and 20,000:1.

16. Process according to claim 15, **characterised in that** the molar ratio of N-benzyl-N-methyl-2-amino-m-hydroxyacetophenone hydrochloride **1** to the rhodium catalyst in the asymmetric hydrogenation is 10,000:1.

17. Process according to one of the preceding claims 1 to 16, **characterised in that** the rhodium catalyst for the asymmetric hydrogenation is used as a pre-prepared solution.

18. Process according to one of the preceding claims 1 to 16, **characterised in that** the rhodium catalyst for the asymmetric hydrogenation is produced *in situ.*

19. Process according to one of the preceding claims 1 to 18, **characterised in that** the reaction time for the asymmetric hydrogenation is between 2 and 8 hours.

20. Process according to the preceding claim 19, **characterised in that** the reaction time for the asymmetric hydrogenation is between 4 and 6 hours.

21. Process according to claim 20, **characterised in that** the reaction time for the asymmetric hydrogenation is 4 hours.

22. Process according to one of the preceding claims 1 to 21, **characterised in that**, after the reaction has ended, the reaction solution of the asymmetric hydrogenation according to step a of claim 1 is combined with activated charcoal and a palladium chloride solution without working up and subjected to a pressure of 2 bar with hydrogen and then the reaction product is isolated.

23. Process according to one of the preceding claims 1 to 21, **characterised in that** after the asymmetric hydrogenation according to reaction step a of claim 1 has ended the N-benzyl-L-phenylephrine **2** formed is isolated as a crude product, this is then dissolved in water at a pH in the range from 5-7 and palladium charcoal is added, the solution is then subjected to a pressure of 1 to 5 bar with hydrogen and finally the reaction product is isolated.

## Revendications

1. Procédé de préparation du chlorhydrate de L-phényléphrine 3 **caractérisé en ce que**, à partir du chlorhydrate de N-benzyl-N-méthyl-2-amino-m-hydroxyacétophénone prochiral 1 comme produit de départ,
dans une première étape
a. on conduit une hydrogénation asymétrique avec [Rh(COD)Cl]₂ et un ligand phosphine bidenté chiral comme système catalytique
et dans une seconde étape réactionnelle
b. on conduit une débenzylation réductrice avec le palladium et l'hydrogène.

2. Procédé selon la revendication 1 **caractérisé en ce que** le ligand est la (2R,4R)-4-(dicyclohexylphosphino)-2-(diphénylphosphino-méthyl)-N-méthylaminocarbonylpyrrolidine.

3. Procédé selon la revendication 1 **caractérisé en ce que** le ligand est la (2R,4R)-4-(dicyclohexylphosphino)-2-(diphénylphosphino-méthyl)-N-méthylaminocarbonyl-pyrrolidine liée à un polymère.

4. Procédé selon l'une des revendications 1 à 3 **caractérisé en ce que** l'hydrogénation asymétrique est conduite dans un domaine de température de 40°C à 100°C.

5. Procédé selon la revendication 4 **caractérisé en ce que** l'hydrogénation asymétrique est conduite dans un domaine de température de 40°C à 60°C.

6. Procédé selon la revendication 5 **caractérisé en ce que** l'hydrogénation asymétrique est conduite dans un domaine de température de 50°C à 55°C.

7. Procédé selon l'une des revendications 1 à 6 précédentes **caractérisé en ce que** l'hydrogénation asymétrique est conduite sous une pression de plus de 1 bar à 100 bar.

8. Procédé selon la revendication 7 **caractérisé en ce que** l'hydrogénation asymétrique est conduite sous une pression de 10 bar à 50 bar.

9. Procédé selon la revendication 8 **caractérisé en ce que** l'hydrogénation asymétrique est conduite sous une pression de 20 bar.

10. Procédé selon la revendication 9 **caractérisé en ce que** l'hydrogénation asymétrique est conduite dans un solvant protique.

11. Procédé selon l'une des revendications 1 à 10 précédentes **caractérisé en ce que** l'hydrogénation asymétrique est conduite dans un alcanol en C₁-C₈ ramifié ou non ramifié comme solvant.

12. Procédé selon la revendication 11 **caractérisé en ce que** l'hydrogénation asymétrique est conduite dans le méthanol, l'éthanol, le n-propanol et/ou l'isopropanol comme solvant.

13. Procédé selon l'une des revendications 10 à 12 précédentes **caractérisé en ce que** le solvant pour l'hydrogénation asymétrique contient de l'eau.

14. Procédé selon l'une des revendications 1 à 13 précédentes **caractérisé en ce que** le rapport molaire du chlorhydrate de N-benzyl-N-méthyl-2-amino-m-hydroxyàcétophénone 1 au catalyseur au rhodium dans l'hydrogénation asymétrique est de 5000:1 à 100000:1.

15. Procédé selon la revendication 14 précédente **caractérisé en ce que** le rapport molaire du chlorhydrate de N-benzyl-N-méthyl-2-amino-m-hydroxyacétophénone 1 au catalyseur au rhodium dans l'hydrogénation asymétrique est de 5000:1 à 20000:1.

16. Procédé selon la revendication 15 **caractérisé en ce que** le rapport molaire du chlorhydrate de N-benzyl-N-méthyl-2-amino-m-hydroxyacétophénone 1 au catalyseur au rhodium dans l'hydrogénation asymétrique est 10000:1.

17. Procédé selon l'une des revendications 1 à 16 précédentes **caractérisé en ce que** le catalyseur au rhodium pour l'hydrogénation asymétrique est utilisé sous forme de solution préparée au préalable.

18. Procédé selon l'une des revendications 1 à 16 précédentes **caractérisé en ce que** le catalyseur au rhodium pour l'hydrogénation asymétrique est produit *in situ*.

19. Procédé selon l'une des revendications 1 à 18 précédentes **caractérisé en ce que** la durée de la réaction pour l'hydrogénation asymétrique est de 2 à 8 heures.

20. Procédé selon la revendication 19 précédente **caractérisé en ce que** la durée de la réaction pour l'hydrogénation asymétrique est de 4 à 6 heures.

21. Procédé selon la revendication 20 **caractérisé en ce que** la durée de la réaction pour l'hydrogénation asymétrique est de 4 heures.

22. Procédé selon l'une des revendications 1 à 21 précédentes **caractérisé en ce que** la solution réactionnelle de l'hydrogénation asymétrique après l'étape a de la revendication 1 est additionnée de charbon actif et d'une solution de chlorure de palladium après la fin de la réaction sans traitement et est exposée à de l'hydrogène à une pression de 2 bar, après quoi le produit réactionnel est isolé.

23. Procédé selon l'une des revendications 1 à 21 précédentes **caractérisé en ce que**, après la fin de l'hydrogénation asymétrique, après l'étape réactionnelle a de la revendication 1, la N-benzyl-L-phényléphrine 2 formée est isolée sous forme de produit brut, celui-ci est ensuite dissous dans l'eau à un pH dans un domaine de 5-7 et additionné de palladium-charbon, puis la solution est exposée à de l'hydrogène à une pression de 1 à 5 bar et enfin le produit réactionnel est isolé.
